# EUROPEAN PATENT SPECIFICATION

(11) **EP 2 842 562 B1**
(45) Date of publication and mention of the grant of the patent: **27.04.2016**
(21) Application number: 14194820.8
(22) Date of filing: 30.03.2011
(51) Int. Cl.: A61K 33/24, A61P 13/12, A61P 9/10

(54) **Tetrathiomolybdate for use in the therapy of ischemia-reperfusion injury of the kidney**
Tetrathiomolybdat zur Verwendung in der Therapie von Ischämie-Reperfusionsverletzung der Niere
Tétrathiomolybdate pour l'utilisation dans la thérapie de la lésion de reperfusion ischémique du rein

(30) Priority: 30.03.2010 GB 201005394
(43) Date of publication of application: 04.03.2015
(62) Divisional of application: 11716626.4
(73) Proprietor: Magnus Oxygen Limited, London WC1R 4AG (GB)
(72) Inventor: Singer, Mervyn, London WC1E 6JF (GB); Martin, John Francis, London WC1E 6FJ (GB); Dyson, Alex Peter, London WC1E 6FJ (GB)
(74) Representative: Gill Jennings & Every LLP

(56) References cited:
- WO-A1-03/075910
- US-A1- 2004 009 237
- US-B1- 6 703 050
- REDMAN BRUCE G ET AL: "Phase II trial of tetrathiomolybdate in patients with advanced kidney cancer.", CLINICAL CANCER RESEARCH : AN OFFICIAL JOURNAL OF THE AMERICAN ASSOCIATION FOR CANCER RESEARCH MAY 2003, vol. 9, no. 5, May 2003 (2003-05), pages 1666-1672, XP002733763, ISSN: 1078-0432
- WANG WEIWEI ET AL: "Netrin-1 and kidney injury. I. Netrin-1 protects against ischemia-reperfusion injury of the kidney", AMERICAN JOURNAL OF PHYSIOLOGY - RENAL PHYSIOLOGY, vol. 294, no. 4, April 2008 (2008-04), pages F739-F747, XP002733764,

## Description

### Field of the Invention

This invention relates to therapy, and in particular to the use of a known compound in the treatment of patients that have undergone severe injury or are in intensive care.

The present invention is defined in the appended claims. Subject matters which are not encompassed by the scope of the claims do not form part of the present invention.

### Background of the invention

Experimental evidence suggests that hypothermia may be beneficial in shock by reducing organ metabolism and increasing the tolerance to ischaemia. Wu et al., J Trauma 2002;53:654-62, compared regional (gut) and systemic hypothermia on survival in a rat haemorrhage model. They found that inducing systemic hypothermia increased 72 h survival time (100%) compared with regional hypothermia (25%) and normothermia (0%). In a follow-up study, the same group, in Wu et al., Crit Care Med 2003;31 :195-202, showed that hepatic injury was reduced.

Childs et al., J Trauma 2005;58:271 -7 show that hypothermia protects against microvascular barrier dysfunction and reactive oxygen species production. Ning et al., Am J Physiol Heart Circ Physiol 2003;285:H212-H219, showed improved myocardial performance in isolated rabbit hearts subjected to hypoxia and reoxygenation during hypothermia compared with normothermic controls. Those under hypothermic conditions recovered better in terms of decreased coronary flow, oxygen consumption and developed pressure.

In a rodent model of experimental sepsis induced by caecal ligation and puncture, survival time was inversely proportional to body temperature from 32-42°C; see L'Her et al., Crit Care Med 2006;34:2621 -3. The utility of hypothermia has also been demonstrated clinically. Hypothermic circulatory arrest is used in some forms of vascular surgery such as aortic arch repair to decrease metabolism and protect against cerebral ischaemia; see Haverich and Hagl., J Thorac Cardiovasc Surg 2003;125:460-2.

In human sepsis associated with acute respiratory distress syndrome, a subset of patients was subjected to hypothermia as a 'last resort'; hypothermia dramatically decreased survival compared with normothermic septic patients (100 vs 67%); see Villar and Slutsky, Resuscitation 1993;26:183-92.

Tetrathiomolybdate (TTM) is known as a therapeutic agent. Brewer et al, Arch Neurol 2006; 63:521 -7, discloses that ammonium TTM can be used to treat Wilson's disease, and that it preserves neurological function in patients who present with neurologic disease.

Brewer et al, Clin. Cancer Res 2000; 6:1 -10, reports that TTM may be suitable in therapy of metastatic disease. Its utility apparently derives from its anti-copper activity.

### Summary of the invention

According to the disclosure TTM is for use in therapy where reduced core temperature is desirable. The therapy is thus, for example, potentially beneficial in cases of shock such as severe hypoxemia and haemorrhage, trauma (e.g. head injury), in reperfusion injury conditions (such as haemorrhage-reperfusion injury and in elective vascular and cardiac surgery surgery involving interruption and re-institution of blood flow). This treatment may be applicable both in hospital or en route, e.g. in an ambulance. Clinical applications of particular interest are those surrounding ischaemia-reperfusion injury conditions such as those involving kidney.

This discovery is based on a showing that, during intravenous infusion of TTM into awake rats, there was a reduction in metabolism reflected by a decrease in oxygen consumption and carbon dioxide production. Notably, conscious level was maintained in the awake animals so this effect was not due to sedation. In anaesthetised rats administration of TTM induced a fall in core temperature. Importantly, it was also observed that TTM ablates the hyperthermic response to endotoxin and even causes hypothermia when administered at 20 mg/kg in endotoxaemic animals.

### Brief Description of the Drawings

Figure 1: Metabolic effects of TTM. Data show change in oxygen consumption and carbon dioxide production from baseline values.
Figure 2: Core temperature at the beginning and end of an experiment where TTM was administered at increasing doses every 20 minutes.
Figure 3: Protocol for endotoxaemia experiments. ETX is endotoxin, TTM is tetrathiomolybdate. Fluids: a 50:50 mix of colloid and crystalloid plus glucose. TTM was diluted in normal saline and administered as a 4 ml/kg bolus.
Figure 4: Core temperature during endotoxaemia. TTM or vehicle was administered 60 minutes following the onset of endotoxaemia.

### Description of the invention

TTM may be used as such or in the form of a pharmaceutically acceptable salt. Salts include pharmaceutically acceptable salts, for example acid addition salts derived from inorganic or organic acids, such as hydrochlorides, hydrobromides, p-toluenesulphonates, phosphates, sulphates, perchlorates, acetates, trifluoroacetates, propionates, citrates, malonates, succinates, lactates, oxalates, tartrates and benzoates.

Salts may also be formed with bases. Such salts include salts derived from inorganic or organic bases, for example alkali metal salts such as magnesium or calcium salts, and organic amine salts such as morpholine, piperidine, dimethylamine or diethylamine salts. A particular salt is ammonium TTM.

A typical dosage is 2 to 20 mg/kg, administered one or more times per day or by continuous infusion. The drug is preferably administered via the intravenous route. It will be understood, however, that the specific dose level for any particular patient will depend upon a variety of factors including the age, body weight, general health, sex, diet, time of administration, drug combination and the severity of the particular condition undergoing therapy.

A pharmaceutical composition containing the active ingredient may be in any suitable form, for example aqueous or non-aqueous solutions or suspensions, dispersible powders or granules, transdermal or transmucosal patches, creams, ointments or emulsions.

The pharmaceutical composition may be in the form of a sterile injectable aqueous or non-aqueous (e.g. oleaginous) solution or suspension. The sterile injectable preparation may also be in a sterile injectable solution or suspension in a non-toxic parenterally-acceptable diluent or solvent, for example as a solution in 1,3-butanediol. Among the acceptable vehicles and solvents that may be employed are water, phosphate buffer solution, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables. Suspensions may be formulated according to the known art using those suitable dispersing or wetting agents and suspending agents which have been mentioned elsewhere.

Aqueous suspensions contain the active ingredient in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents such as a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Non-aqueous (i.e. oily) suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are known.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally occurring gums, for example gum acacia or gum tragacanth, naturally occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate.

The active agent may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical delivery, transdermal and transmucosal patches, creams, ointments, jellies, solutions or suspensions may be employed. For sub-lingual delivery, fast dissolving tablet formulations may be used, as well as a number of the presentations described above. For oral administration, the drug may be administered as tablets, capsules or liquids.

The following studies provide evidence on which the present invention is based.

### Study 1

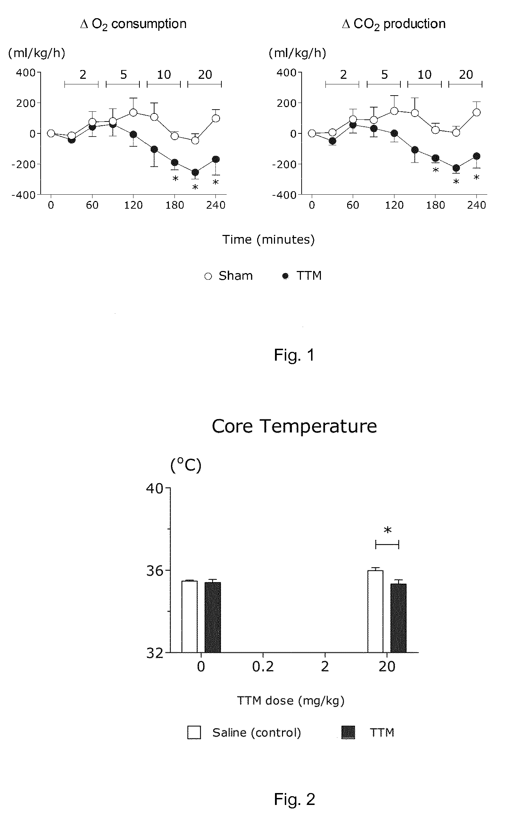

Male Wistar rats (300 g) were anaesthetised and instrumented with a venous line for drug administration. The animals were placed into a metabolic cart and allowed to recover from anaesthesia. After 2 h, TTM was administered i.v. hourly at increasing doses (2, 5, 10 and 20 mg/kg). Oxygen consumption and carbon dioxide production were continually monitored for the duration of the experiment. TTM induced a clear drop in oxygen consumption and carbon dioxide production at 10 and 20 mg/kg (Fig. 1), compared to time-matched control animals (sham) receiving only vehicle (saline).

### Study 2

Animals were anaesthetised and instrumented as above. Under continuous anaesthesia, TTM was administered every 20 minutes at increasing doses (0.2, 2 and 20 mg/kg). Core temperature was measured at the beginning of the experiment (i.e. before TTM administration) and 20 minutes after the last dose of the drug (20 mg/kg). A decrease in core temperature was introduced by TTM (Fig. 2), compared to animals receiving vehicle (saline).

### Study 3

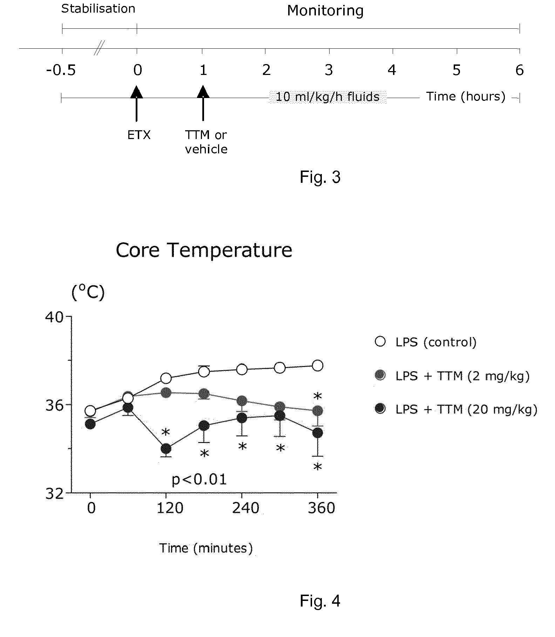

Animals received a venous line for drug administration under anaesthesia. Endotoxaemia (which typically causes an increase in core temperature) was induced by intravenous administration of lipopolysaccharide (20 mg/kg, *Klebsiella pneumonia).* TTM or vehicle was administered 1 h following the onset of endotoxaemia (Fig. 3; protocol). Administration of TTM ablated the hyperthermic response to endotoxin observed in control animals and induced significant hypothermia at the highest dose (20 mg/kg) (Fig. 4).

## Claims

1. Tetrathiomolybdate (TTM) for use in the therapy of ischemia-reperfusion injury of the kidney.

2. TTM for use according to claim 1, wherein the subject of therapy is in intensive care.

3. TTM for use according to claim 1 or claim 2, wherein TTM is administered intravenously.

4. TTM for use according to any preceding claim, wherein TTM is administered by continuous infusion at a dosage of 2 to 20 mg/kg.

## Patentansprüche

1. Tetrathiomolybdat (TTM) zur Verwendung bei der Therapie einer Ischämie-Reperfusionsschädigung der Niere.

2. TTM zur Verwendung gemäß Anspruch 1, wobei das Subjekt der Therapie auf der Intensivstation ist.

3. TTM zur Verwendung gemäß Anspruch 1 oder Anspruch 2, wobei TTM intravenös verabreicht wird.

4. TTM zur Verwendung gemäß einem vorhergehenden Anspruch, wobei TTM mittels Dauerinfusion mit einer Dosierung von 2 bis 20 mg/kg verabreicht wird.

## Revendications

1. Tétrathiomolybdate (TTM) pour utilisation dans le traitement de la lésion de reperfusion ischémique du rein.

2. TTM pour utilisation selon la revendication 1, où le sujet du traitement est en unité de soins intensifs.

3. TTM pour utilisation selon la revendication 1 ou la revendication 2, où le TTM est administré par voie intraveineuse.

4. TTM pour utilisation selon n'importe quelle revendication précédente, où le TTM est administré par perfusion continue à une posologie de 2 à 20 mg/kg.
